(19) Europäisches Patentamt
European Patent Office
Office européen des brevets

(11) Publication number: **0 119 840**
**A1**

(12) **EUROPEAN PATENT APPLICATION**

(21) Application number: **84301770.8**

(22) Date of filing: **15.03.84**

(51) Int. Cl.³: **C 07 D 317/36**, C 07 D 317/38, C 07 C 68/00, C 07 C 69/00

(30) Priority: **18.03.83 GB 8307613**

(43) Date of publication of application: **26.09.84**
**Bulletin 84/39**

(84) Designated Contracting States: **BE DE FR GB IT NL**

(71) Applicant: **BP Chemicals Limited, Belgrave House 76 Buckingham Palace Road, London, SW1W 0SU (GB)**

(72) Inventor: **Green, Michael James, BP Chemicals Limited Saltend, Hedon Hull HU12 8DS (GB)**

(74) Representative: **Crack, Richard David et al, c/o The British Petroleum Company plc Patents Division Chertsey Road, Sunbury-on-Thames Middlesex, TW16 7LN (GB)**

(54) **Preparation of carbonates.**

(57) Alkylene carbonates and optionally dialkyl carbonates are prepared by reaction of an alkylene oxide with carbon dioxide in the presence of an alcohol. In addition there is added to the reaction mixture a trivalent phosphorus containing compound as catalyst. In an embodiment, the alkylene carbonate is further transesterified with the alcohol to produce a dialkyl carbonate and an alkylene glycol.

EP 0 119 840 A1

ACTORUM AG

# PREPARATION OF CARBONATES

This invention relates to a process for the preparation of carbonate esters, more particularly to the preparation of alkylene carbonates and optionally dialkyl carbonates by the reaction of carbon dioxide with an alkylene oxide in the presence of an alcohol.

The reaction of an alkylene oxide such as ethylene oxide with carbon dioxide in the presence of a tertiary phosphine as catalyst to form an alkylene carbonate has been previously reported in Japanese Patent No 73 22 702 (Chemical Abstracts 1973, Vol 79 at page 284).

Further, the reaction of an alkylene oxide with carbon dioxide in the presence of, as catalyst, a mixture of a protic substance such as water or an alcohol and a nitrogenous base such as triethylamine, to form an alkylene carbonate has been described in UK Patent No 1,485,925.

It has now been found that the reaction between carbon dioxide and an alkylene oxide can be effected in the presence of an alcohol using as catalyst a trivalent phosphorus containing compound and that the reaction is very much faster than that described in the above Japanese patent where the alcohol is absent.

According to the present invention a process for the preparation of alkylene carbonates and optionally dialkyl carbonates comprises reacting an alkylene oxide with carbon dioxide in the presence of an alcohol and, as catalyst, a trivalent phosphorus-containing compound.

The trivalent phosphorus containing compound can be a tertiary phosphine e.g. a trialkyl or a triaryl phosphine and may contain

more than one trivalent phosphorus atom.

Suitable phosphines are of formula : $R_1$ $R_2$ P $R_3$

where $R_1$, $R_2$ and $R_3$, which can be the same or different, are hydrogen, or substituted or unsubstituted $C_1$ to $C_{10}$ alkyl, aryl, alkaryl, or aralkyl groups. Also one or more of the $R_1$, $R_2$ and $R_3$ groups can contain a tertiary phosphorus atom. Insoluble or heterogenised phosphines can also be used. Preferred phosphines of this type are those where the phosphine is bonded to a polymeric backbone by means of one or more of the groups $R_1$, $R_2$ and $R_3$.

The alcohol can be a primary aliphatic alcohol, for example methanol, ethanol, propanol, butanol or polyols such as ethylene or propylene glycol.

Conveniently the amount of alcohol is at least the molar equivalent of the phosphine, and is preferably sufficient to dissolve substantially all the reactants.

The alkylene oxide used is conveniently a 1,2-alkylene oxide. Preferred examples of such alkylene oxides are ethylene oxide, propylene oxide and 1,2-butylene oxide.

Preferably the reaction is effected under the following conditions:

temperature from 60 to 150°C

pressure in excess of 5 bars

According to another aspect of the present invention a process for the transesterification of an alkylene carbonate comprises reacting the alkylene carbonate prepared as hereinbefore described with an alcohol under transesterification conditions to form an alkylene glycol and a carbonate ester of the alcohol.

Suitable catalysts for the transesterification reaction are those employed for the reaction of the carbon dioxide with the alkylene oxide to form the alkylene carbonate.

One way in which the reaction can be performed is to first react the carbon dioxide with the alkylene oxide to form the alkylene carbonate in the presence of for example an approximately

stoichiometric amount of the alcohol and to continue the reaction to effect the transesterification.

The invention is illustrated by the following examples in which the reactants and products (except the carbon dioxide) were predominantly in the liquid phase.

Example 1 - Preparation of propylene carbonate and dimethyl carbonate

A 100 ml high pressure stirred autoclave was charged with 15 g of propene oxide, 15 g of methanol, and 1.5 g of triphenylphosphine. The autoclave was sealed and flushed twice with carbon dioxide, following which it was pressurised to 21 bar with carbon dioxide and finally heated to 130°C with stirring (1200 rpm). After 3 h, the autoclave was cooled to 15°C. Analysis of the liquid product by gas chromatography showed a propene oxide conversion of 89% to propylene carbonate and a 8% conversion of methanol to dimethyl carbonate.

Example 2 - Preparation of propylene carbonate

Example 1 was repeated except that 15 g of ethanol was used in place of methanol. Analysis of the liquid product showed a propene oxide conversion of 88% with a selectivity to propylene carbonate of 98%.

Example 3 - Preparation of butylene carbonate and dimethyl carbonate

Example 1 was repeated except that 15 g of butene oxide was used in place of propene oxide. Analysis of the liquid product showed a butene oxide conversion of 87% with selectivities to butylene carbonate and glycol of 86 and 6% respectively (and a 5% conversion of methanol to dimethyl carbonate).

Example 4 - Preparation of propylene carbonate and dimethyl carbonate

Example 1 was repeated except that the reaction time was increased to 6 h. Analysis of the liquid product showed a quantitative conversion of propene oxide with selectivities to propylene carbonate and glycol of 72 and 19% respectively (and an 18% conversion of methanol to dimethyl carbonate).

Example 5 - Preparation of ethylene carbonate and dimethyl carbonate

Example 1 was repeated except that 15 g of ethylene oxide was used in place of propene oxide. Analysis of the liquid product showed a quantitative conversion of ethylene oxide with selectivities to ethylene carbonate and ethylene glycol of 64 and 29% respectively (and a 33% conversion of methanol to dimethyl carbonate).

Example 6 - Preparation of propylene carbonate and dimethyl carbonate

Example 1 was repeated except that 2.3 g of bis(diphenylphosphino) ethane was used as a catalyst in place of triphenylphosphine. Analysis of the liquid product showed a quantitative conversion of propene oxide with selectivities to propylene carbonate and glycol of 76 and 18% respectively (and a 15% conversion of methanol to dimethyl carbonate).

Example 7 -Preparation of propylene carbonate and dimethyl carbonate

Example 1 was repeated except that 1.2 g of tri-n-butyl phosphine was used as a catalyst in place of triphenyl phosphine. Analysis of the liquid product showed a quantitative conversion of propene oxide with selectivities to propylene carbonate and glycol of 71 and 18% respectively (and a 16% conversion of methanol to dimethyl carbonate).

Comparative Test A

Example 1 was repeated in the absence of the methanol and using 30 g of propene oxide. Analysis of the liquid product showed a propene oxide conversion of 7% to propylene carbonate.

This test illustrates that the reaction carried out in the absence of methanol gives only a very low conversion of propene oxide (7%) as compared with 89% according to the present invention.

Comparative Test B

Example 1 was repeated in the absence of triphenylphosphine. Analysis of the liquid product showed a propene oxide conversion of less than 1% to propylene carbonate.

0119840

The above described invention has the following advantage:

As compared with amines, phosphines have a lower volatility and hence losses in use are smaller.

Claims:

1. A process for the preparation of alkylene carbonates and optionally dialkyl carbonates characterised in that an alkylene oxide is reacted with an alcohol and carbon dioxide in the presence of, as catalyst, a trivalent phosphorus-containing compound.

2. A process as claimed in claim 1 characterised in that the trivalent phosphorus compound is of formula :

$$\begin{matrix} R_1 & \\ R_2 & P \\ R_3 & \end{matrix}$$

where $R_1$, $R_2$ and $R_3$, which can be the same or different, are hydrogen or substituted or unsubstituted $C_1$ to $C_{10}$ alkyl, aryl, alkaryl, or aralkyl groups.

3. A process as claimed in claim 2 characterised in that the phosphine is selected from the group containing triphenyl phosphine, tributyl phosphine or bis(diphenyl phosphino)ethane.

4. A process as claimed in claim 1 characterised in that the alcohol is a primary aliphatic alcohol.

5. A process as claimed in claim 4 characterised in that the alcohol is methanol.

6. A process as claimed in claim 1 or claim 5 characterised in that the alkylene oxide is either ethylene or propylene oxide.

7. A process as claimed in claim 1 or 2 characterised in that the alkylene carbonate is reacted with the alcohol under transesterification conditions to form an alkylene glycol and a dialkyl carbonate.

8. A process as claimed in claim 1 or 7 characterised in that the pressure is in the range 5 to 100 bars and the temperature from 50 to 150°C.

9. Alkylene and dialkyl carbonates characterised in that such materials are produced by a process as claimed in any one of the preceding claims.

0119840

European Patent Office

# EUROPEAN SEARCH REPORT

Application number

EP 84 30 1770

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| D,Y | CHEMICAL ABSTRACTS, vol. 79, 1973, page 284, no. 146007k, Columbus, Ohio, US; & JP - A - 73 22 702 (MITSUI PETROCHEMICAL INDUSTRIES, LTD.) 07-07-1973<br>* Abstract *<br>--- | 1-3,6 | C 07 D 317/36<br>C 07 D 317/38<br>C 07 C 68/00<br>C 07 C 69/00 |
| D,Y | GB-A-1 485 925 (ANIC)<br>* Pages 1-3 *<br>----- | 1-3,6 | |
| | | | TECHNICAL FIELDS SEARCHED (Int. Cl. 3) |
| | | | C 07 D 317/00 |

The present search report has been drawn up for all claims

| Place of search | Date of completion of the search | Examiner |
|---|---|---|
| THE HAGUE | 21-06-1984 | FRANCOIS J.C.L. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document